Europäisches Patentamt

European Patent Office

Office européen des brevets

(19)

(11) Publication number : **0 473 398 A1**

(12) **EUROPEAN PATENT APPLICATION**

(21) Application number : **91307838.2**

(22) Date of filing : **28.08.91**

(51) Int. Cl.⁵ : **C07D 301/03, C07D 303/48**

(30) Priority : **29.08.90 US 573070**

(43) Date of publication of application :
**04.03.92 Bulletin 92/10**

(84) Designated Contracting States :
**AT BE CH DE DK ES FR GB GR IT LI LU NL SE**

(71) Applicant : **E.I. DU PONT DE NEMOURS AND COMPANY**
**1007 Market Street**
**Wilmington Delaware 19898 (US)**

(72) Inventor : **Lawson, James Robert**
**10 Hickory Lane**
**Marietta, OH 45750 (US)**

(74) Representative : **Jones, Alan John et al**
**CARPMAELS & RANSFORD 43 Bloomsbury Square**
**London, WC1A 2RA (GB)**

(54) **Two-liquid-phase epoxidation of hexafluoropropylene at low PH.**

(57) A process for converting hexafluoropropylene to its epoxide by agitation at reaction conditions that maintain two liquid phases - a pH 7.5-9.6 aqueous phase containing hypohalite ions and a reaction phase comprising an organic liquid in which the a phase transfer catalyst is soluble and in which there is a higher affinity for the hexafluoropropylene than for its epoxide, most of which is present in the vapor phase.

EP 0 473 398 A1

## FIELD OF INVENTION

The present invention relates to a process for converting hexafluoropropylene (HFP) to hexafluoropropylene epoxide (HFPO).

## BACKGROUND

Hexafluoropropylene epoxide (HFPO) is a versatile intermediate which can be converted to inert oils and to vinyl monomers, particularly perfluoromonomers. It has been made from hexafluoropropylene (HFP) by several processes, most of which have selectivity[1] in the range of 60-80%. Millauer, Chem Ing Tech 52, 53-55 (1980) reports an electrochemical process which gives a selectivity of 90%, but the current yield is only about 65% and the conversion[2] of HFP is about 65-70%. Kolenko et al, Izv. Akad. Nauk, SSR, Ser. Khim. (1979) No. 11, pp. 2509-2512, made HFPO in 52% selectivity in a one-liquid-phase system containing acetonitrile and aqueous NaOCl and no phase transfer catalyst.

Of particular note is the process taught in Ikeda et al. (Ep 64293 and U.S. 4,902,810). It is a hypochlorite oxidation employing two liquid phases, using a phase transfer catalyst. The best selectivity among 55 examples was in Example 1, carried out in a 50 milliliter (ml) pressure bottle. That example stated a 96% conversion of HFP and an HFPO selectivity of 84% were obtained.

The main problem Ikeda et al. were trying to solve was to find a process for converting HFP to HFPO in very high conversion, with as high a selectivity as possible. This was presumably because they wanted to avoid having to separate HFP from HFPO. However, HFP and HFPO can be separated by extractive distillation and, in some reactions, HFPO can be used without separating it from HFP, which is inert in many of the reactions of HFPO.

The problem of the present invention is to obtain the maximum selectivity at moderate conversion of HFP, using a minimum amount of phase transfer catalyst, and minimizing environmental problems and pH control problems.

Ikeda et al. collect HFPO in the organic phase of a two-liquid-phase system, while the present invention removes HFPO from the reactor as it is formed. Further, the present invention teaches how to select an organic liquid for use as one of the two liquid phases in such a way that HFPO is less soluble in that liquid than is HFP, thus protecting HFPO from attack by hypochlorite more effectively than the process of Ikeda et al.

Ikeda et al. named 25 specific organic liquids. Their examples used F-113 ($CF_2ClCFCl_2$), perfluorodimethyl cyclobutane, $CCl_4$, $CHCl_3$, $CH_2Cl_2$, $ClCH_2CH_2Cl$, n-hexane, diisopropyl ether, and a l benzene/9 hexane mixture. None of these liquids is all-aromatic. The organic liquids for use in the present invention are selected by the partition coefficient described below, which is designed to provide more HFP than HFPO in the organic liquid phase and more HFPO than HFP in the vapor phase at reaction conditions. Many of the best liquids are aromatic.

Ikeda et al., in both EP 64293 and its partial equivalent U.S. 4,902,810, are not clear as to the pH or the amount of added base preferred in their claimed process. They do, however, show in Table I that the sodium hypochlorite solution is charged at high pH. In the prosecution history of U.S. 4,902,810, they state that good reaction results cannot be obtained in their reaction using a phase transfer catalyst when the pH in the aqueous phase is low.

In the prosecution of U.S. 4,902,810, Ikeda et al. abandoned coverage of processes using low pH, requiring that the reaction be carried out "in the presence of an inorganic base". In the present invention, pH is in the range 7.5-9.6, preferably 7.8-9.0, and most preferably 8.0-8.5, ranges abandoned by Ikeda et al. in U.S. 4,902,810.

Example 1 of U.S. 4,902,810 and most of its other examples used $CF_2ClCFCl_2$ as the organic solvent. The use of such an organic solvent is undesirable because this chlorofluorocarbon is the type of compound believed to cause problems in the ozone layer of the atmosphere.

Example 1 of U.S. 4,902,810 and nearly all its other examples use at least twice the stoichiometric amount of hypochlorite, with the result that much hypochlorite is wasted and a highly alkaline oxidizing waste stream must be disposed of, creating a safety and environmental problem.

In the present invention, the use of chlorofluorocarbons is easily avoided. Also, the amount of waste hypochlorite and caustic is sharply reduced.

---

[1]Selectivity is defined as grams of product produced divided by grams of starting material consumed, expressed as a percentage.
[2]Conversion is defined as grams of starting material converted divided by grams of starting material charged, expressed as a percentage.

The selectivity to HFPO is, in the better examples of the present invention, comparable to that of the best example of U.S. 4,902,810.

## SUMMARY OF THE INVENTION

The present invention is a process for converting hexafluoropropylene (HFP) to its epoxide (HFPO) by agitation of a system of two liquid phases --an aqueous phase and a reaction phase -- in the presence of a phase transfer catalyst for a sufficient time to convert a substantial part of the HFP to HFPO.

The aqueous phase is a solution of a water-soluble metal hypohalite, containing 0.001 to 25% available halogen by weight, at a pH that provides maximum selectivity at the highest attainable conversion. The pH is 7.5-9.6; conversion can be increased and phase transfer catalyst concentration can be decreased by operating at this pH range contrary to the suggestions of U.S. 4,902,810.

The preferred hypochlorite is sodium hypochlorite.

The reaction phase comprises an organic liquid, in which the catalyst is soluble and in which HFP is more readily soluble than HFPO at reaction conditions. It contains sufficient HFP to provide a reasonable reaction rate. The selection of an appropriate organic liquid is carried out by measuring a partition coefficient as described below. Preferably, the organic liquid is toluene.

The phase transfer catalyst can be any known phase transfer catalyst that is resistant to oxidation and halogenation at reaction conditions. It should be soluble in the reaction phase. The literature, including U.S. 4,902,810, teaches several classes of catalysts which are stated to be functional. Quaternary ammonium salts are preferred because of availability, freedom from toxicity, and cost.

Reaction conditions should be such that two liquid phases are maintained as the reaction progresses. It is particularly important that the reaction phase or organic liquid not be oxidized or halogenated to a compound in which HFPO would be equally as soluble as is HFP.

The process may be batch or continuous. The reaction temperature should be high enough that no component is substantially a solid. The reaction pressure should be such that two and only two liquid phases are present.

The perfluoroolefin epoxide is recovered from the reaction mixture that results, preferably about as fast as it is formed.

## DETAILS OF THE INVENTION

It is particularly important that the reaction system of the present invention contain two liquid phases. While not wishing to be inappropriately limited, it is believed that the phase transfer catalyst helps transport the oxidant from the aqueous phase into the reaction phase where it reacts with the olefin. The epoxide product, being less soluble than the reactant perfluoroolefin in the reaction phase, separates into a gaseous epoxide phase where it is protected from over-oxidation by the non-volatile oxidant until the gas phase is removed.

The oxidant can be any hypochlorite, with sodium hypochlorite being the most readily available. The hypochlorite concentration in the reaction may be 0.001 to 25 wt % (as chlorine) in the aqueous phase depending upon which organic liquid is used and the conditions of reaction. It is important not to substantially alter the ability of the organic liquid to dissolve the HFP more extensively than it dissolves HFPO.

To minimize undesired byproducts from hypochlorite attack on the organic liquid or catalyst and to reduce the amount of hypochlorite present in the aqueous waste, it is desirable to maintain the hypochlorite concentration at a low level, preferably 1.5 wt % or less as chlorine in the aqueous phase. For rate of reaction, a concentration of 0.01 or higher is preferred.

The ratio of hypochlorite to HFP, particularly in continuous reactions, should be limited to a molar ratio of less than 1.2 to 1 in order to minimize attack on organic liquid and catalyst and to minimize hypochlorite content of aqueous waste streams. The molar ratio preferably should be 0.5 to 1 or higher to provide acceptable conversion.

The phase transfer catalyst can be any known phase transfer catalyst that is resistant to oxidation and halogenation under reaction conditions. The literature, including U.S. 4,902,810, teaches several classes of catalysts which are stated to be functional. U.S. 4,902,810, incorporated by reference, discloses a lengthy list of such phase transfer catalysts including those selected from the class consisting of

    a) quaternary ammonium salts,
    b) quaternary phosphonium salts,
    c) quaternary arsonium salts,
    d) sulphonium salts,
    e) onium salts,

f) crown ethers, and

g) lipophilic complexing agents.

The preferred catalysts are organic-soluble quaternary ammonium salts, more preferably coco benzyl bis(betahydroxypropyl) ammonium chloride[3]

The concentration of the phase transfer catalyst or catalysts is not critical. The optimum catalyst concentration depends on the structure of the catalyst and the selection of the organic liquid. Increased catalyst concentration increases reaction rate and conversion. At excessively high catalyst concentrations, however, selectivity decreases. With the coco benzyl bis(betahydroxypropyl) ammonium chloride and toluene used in the examples, a suitable concentration is between 0.001 and 0.2 grams per gram (g/g) HFP, preferably between 0.005 and 0.05 g/g HFP, and most preferably between 0.008 and 0.02 g/g HFP. For each catalyst and organic liquid, routine experimentation will determine the best balance between selectivity and conversion. Sufficient catalyst to achieve the optimum balance should be used.

Many organic liquids which are substantially halogenated are good solvents for both HFP and HFPO are not preferred in the present invention. This has been demonstrated in the case of $CF_2ClCFCl_2$. Certain organic liquids show more affinity for HFP than for HFPO and they are desired for use in this invention. These liquids are generally hydrogen-containing organic liquids, preferably aromatic liquids.

The reaction phase organic liquid or solvent should be resistant to reaction with any component of the system at reaction conditions, particularly if such reaction would alter the preference for dissolving HFP to a greater extent than HFPO.

A simple method of determining this preference is to determine the partition coefficient for the system HFPO-HFP-organic liquid. Determination of the partition coefficient (alpha) is carried out by passing a mixture of HFP and HFPO gas through the organic liquid at atmospheric pressure while agitating vigorously and maintaining a constant temperature. The gas is passed through the liquid long enough to ensure that the liquid is completely saturated with the gases and to let the gas phase composition equilibrate to the feed composition. The organic liquid phase is then sampled and gas chromatography is used to determine the amount of the gaseous components which had been dissolved in the liquid phase. The partition coefficient, alpha, is then calculated by the following equation:

$$\text{alpha} = \frac{([HFPO] / [HFP])\ \text{gas phase}}{([HFPO] / [HFP])\ \text{liquid phase}}$$

An HFP-HFPO partition coefficient greater than 1.0 means that the organic liquid dissolves HFP more readily than it dissolves HFPO. Preferably, the partition coefficient for the present invention should be greater than 1.3, more preferably 1.9 or greater, and most preferably 2.3 or greater at the temperature of operation. Higher partition coefficients suggest that the organic liquid will perform well in the present invention provided it fulfills the other requirements described herein.

Using this method, partition coefficients were determined for a number of organic liquids at -15°C and 0°C, with the results shown below:

| Solvent | Alpha at -15°C | Alpha at 0°C |
|---|---|---|
| FC-75[4] | 1.0 | 1.0 |
| n-decane | 1.6 | 1.3 |
| chlorobenzene | 1.9 | 2.1 |
| dichlorobenzene | 2.0 | 2.1 |
| mesitylene | 2.3 | 2.2 |
| anisole | 2.4 | 2.5 |
| toluene | 2.6 | 2.3 |

[4]Perfluoro(butyl tetrahydrofurane)

For the present invention, toluene is the preferred solvent, but several other aromatic solvents are attractive.

The organic phase must be substantially immiscible or very sparingly miscible with the aqueous phase.

The vapor phase is rich in HFPO, and it also contains some HFP. The HFP:HFPO ratio is higher in the

[3]The term "coco" refers to a mixture of alkyl groups containing 12-18 carbon atoms.

liquid phase than in the vapor phase. Therefore it is preferred to remove the HFPO from the vapor phase rather than from the liquid phase. More specifically, the HFPO is preferably transferred from the organic liquid phase to the vapor phase as HFPO is formed, and the vapor phase is continuously removed from the reactor.

The process of the invention can be run in a variety of reactor configurations including but not limited to batch, semi-batch, continuous back-mixed tank, and continuous plug flow. The type of reactor is not critical.

Reaction temperature is critical only in the sense that two liquid phases and an HFPO-rich vapor phase must be present during the reaction, but it is significant in three ways. First, when using an aqueous medium, the minimum temperature which can be reached without freezing the water is about -25°C, even with maximum addition of NaCl to suppress the freezing point. (Other salts such as $CaCl_2$ suppress the freezing point even more.) Second, increasing the temperature appears to decrease the selectivity. Finally, as stated above, the temperature must be maintained in a region where two liquid phases and an HFPO-rich vapor phase exist. The last two considerations limit maximum temperature to about 60°C. The temperature range for HFP oxidation is preferably from -20° to +15°C, more preferably -15° to +5°C. Operation at the higher end of the preferred range is particularly suited to continuous operation.

Reaction pressure is not critical except that two liquid phases must be present, and the HFPO must exist largely in the vapor phase. Therefore total pressure should be no higher than the sum of the partial pressures of the system components at the particular temperature of operation. The pressure is preferably atmospheric pressure, but operation at slightly higher pressure (up to 0.3-0.5 kg/cm$^2$) is acceptable for continuous operation.

The pH of the system is 7.5-9.6, more preferably 7.8-9.0, most preferably 8.0-8.5. Control of pH becomes more difficult above pH 9.6, and more phase transfer catalyst may be required to effect good conversion of HFP. The use of an added buffer such as $K_2CO_3$ or equivalent is desirable at these pH ranges, as it makes pH control easier. Hypochlorite is also a buffer, and may be used as the sole buffer.

The claimed pH range may involve a different mechanism from operation at pH 10-13 (see JACS 105 7672-7676 (1983)). This reference suggests that ClO·, a free radical, is the reactive intermediate in the reaction under phase transfer catalysis conditions at pH 7.5-9 of hypochlorite with either alkenes, toluene, alkanes, or anisole. These workers did not use any fluoroolefin, certainly not HFP, and the cited reaction temperature, reaction time, and phase transfer catalyst were different from those of the present work. The prior art work with toluene caused almost 10% conversion of toluene, while the present work found less than 1% conversion of toluene at the claimed pH. In any event, operation at the preferred pH range provides good conversion and excellent selectivity even with a low concentration of hypochlorite.

## EXAMPLES

### EXAMPLE 1-11

This series of experiments studying the effect of variables was carried out one after another in a 1 liter jacketed round bottom flask with four vertical indentations which acted as baffles, an agitator, a thermometer, a pH probe, and various feed and exit lines. HFP was fed to the reactor as a gas through a submerged glass tube. Sodium hypochlorite was added continuously to the reactor by pump. As required, an alkaline solution was added periodically to maintain the desired pH.

The only exit stream from this atmospheric pressure reactor was the gas stream. The liquid feeds were allowed to accumulate in the reactor. The gas phase passed out of the top of the reactor and into dry ice-cooled Hoke cylinders for later analysis. The initial charge was 100 milliliter (ml) toluene, 300 g water, 380 g Clorox® bleach, which is NaOCl with about 4.2% available chlorine, and 90 g $K_2CO_3$. HFP was fed at 0.6 g/min until a total of 45 g had been fed, and 3N NaOH was added as needed to control pH. Variables shown in the following table were pH, weight of coco benzyl bis(beta-hydroxypropyl) ammonium chloride phase transfer catalyst, and temperature.

EP 0 473 398 A1

| Ex. | g. catalyst | °C | pH | Conversion | Selectivity |
|---|---|---|---|---|---|
| 1 | 3.0 | 1.6 | 9.5 | 53.2 | 63.7 |
| 2 | 3.0 | 2.0 | 11.5 | 55.0 | 73.2 |
| 3 | 1.0 | 15.6 | 11.5 | 46.4 | 65.9 |
| 4 | 1.0 | 0.4 | 9.5 | 57.5 | 69.0 |
| 5 | 0.5 | 3.6 | 9.6 | 48.3 | 83.5 |
| 6 | 0.5 | 0.8 | 11.4 | 42.5 | 85.9 |
| 7 | 1.0 | 13.8 | 9.4 | 52.3 | 72.0 |
| 8 | 3.0 | 2.5 | 9.6 | 60.8 | 66.5 |
| 9 | 3.0 | 1.3 | 11.4 | 59.6 | 58.6 |
| 10 | 1.0 | 14.5 | 11.4 | 47.8 | 67.7 |
| 11 | 1.0 | -3.2 | 11.5 | 47.5 | 85.3 |
| average for pH 9.4-9.6 | | | | 54.4 | 70.9 |
| average for pH 11.4-11.5 | | | | 49.8 | 72.8 |

These averages show that, contrary to the amendments in view of prior art made during the prosecution of U.S. 4,902,180, there is no superiority for the higher pH. If anything, the lower pH gives higher conversion.

**Claims**

1. A process for converting hexafluoropropylene to hexafluoropropylene epoxide comprising agitating a system of two liquid phases and a hexafluoropropylene/hexafluoropropylene epoxide vapor phase in the presence of a soluble quaternary ammonium salt phase transfer catalyst at a pH controlled between 7.5 and 9.6 at a temperature between -20°C and +15°C, the two liquid phases being:
   a) an aqueous solution of a sufficient amount of metal hypochlorite, and
   b) a reaction phase, the major component of which is an organic liquid, said organic liquid having a hexafluoropropylene/hexafluoropropylene epoxide partition coefficient greater than 1.3 at reaction conditions.

2. The process of Claim 1 wherein the partition coefficient is at least 1.9.

3. The process of Claim 2 wherein the partition coefficient is at least 2.3.

4. The process of Claim 1, 2 or 3 wherein the aqueous phase contains a buffer in addition to the hypochlorite.

5. The process of any one of Claims 1 to 4 wherein the metal hypochlorite is sodium hypochlorite and the organic liquid in the reaction phase is an aromatic compound.

6. The process of any one of Claims 1 to 5 wherein the metal hypochlorite is sodium hypochlorite buffered with an alkali metal carbonate and the organic liquid in the reaction phase is toluene.

7. The process of any one of Claims 1 to 6 wherein the quaternary ammonium salt is coco benzyl bis(betahydroxypropyl) ammonium chloride.

8. The process of Claim 7 in which the catalyst is present in an amount between 0.001 and 0.2 g/g hexafluoropropylene.

9. The process of Claim 8 wherein the catalyst is present in an amount between 0.005 and 0.05 g/g hexafluoropropylene.

6

10. The process of any one of Claims 1 to 9 wherein the mole ratio of metal hypochlorite to hexafluoropropylene is about 0.5/1 to 1.2/1.

11. The process of any one of Claims 1 to 10 wherein the reaction temperature is between -15°C and +5°C.

12. The process of any one of Claims 1 to 11 wherein the pH of the aqueous phase is between 7.8 and 9.0.

13. The process of Claim 12 wherein the pH is between 8.0 and 8.5.

14. The process of any one of Claims 1 to 13 wherein vapor phase HFPO is removed from the reaction mixture as it is produced.

**European Patent Office**

# EUROPEAN SEARCH REPORT

Application Number

| | DOCUMENTS CONSIDERED TO BE RELEVANT | | EP 91307838.2 | |
|---|---|---|---|---|

| Category | Citation of document with indication, where appropriate, of relevant passages | Relevant to claim | CLASSIFICATION OF THE APPLICATION (Int. Cl.5) |
|---|---|---|---|
| D,A | EP - A1 - 0 064 293 (ASAHI KASEI KOGYO KABUSHIKI KAISHA) * Totality * | 1-14 | C 07 D 301/03 C 07 D 303/48 |
| D,A | US - A - 4 902 810 (IKEDA et al.) * Claims * | 1-14 | |

**TECHNICAL FIELDS SEARCHED (Int. Cl.5)**

C 07 D 301/00
C 07 D 303/00

The present search report has been drawn up for all claims

| Place of search | Date of completion of the search | Examiner |
|---|---|---|
| VIENNA | 15-10-1991 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)